# EUROPEAN PATENT APPLICATION

(11) **EP 1 321 532 A1**
(43) Date of publication of application: **25.06.2003**
(21) Application number: 02028282.8
(22) Date of filing: 17.12.2002
(51) Int. Cl.: C12Q 1/68

(54) **Reagent for improved PCR**

(30) Priority: 19.12.2001 EP 01130252
(71) Applicant: Roche Diagnostics GmbH, 68305 Mannheim (DE); F. HOFFMANN-LA ROCHE AG, 4070 Basel (CH)
(72) Inventor: Heindl, Dieter, Dr., 82327 Tutzing (DE); Birkner, Christian, Dr., 82449 Uffing (DE); von der Eltz, Herbert, Dr., 82362 Weilheim (DE)

(57) **Abstract**

The present invention is directed to the use of a poly-hydroxy-aryl-poly-acid with 3-6 ortho-hydroxy-acid moieties as an additive for a nucleic acid amplification reaction. Surprisingly, such an addition results in prevention of nonspecific primer dimer formation and thus in a more specific and sensitive PCR.

## Description

The new invention relates to the field of amplification of nucleic acids. More precisely, the new invention provides compounds and methods for improvements of the Polymerase Chain Reaction

### Prior art background

Commercially available aurintricarboxylic acid (ATA) is known to be a heterogenous polymeric composition consisting of many different components. Such a polymeric composition is known to consist of multiple different low and high molecular weight compounds with varying numbers of ATA monomers (Cushman, M., et al., Journal of Organic Chemistry 57 (1992) 7241-7248; Cushman, M., et al., J Med Chem 34 (1991) 337-42). Moreover, these ATA monomer residues may be derivatized.

Commercially available ATA or fractionated components of commercially available ATA have often been used as additives for improvement of in vitro reactions in the field of molecular biology as well as for in vivo applications.

For example, it has been shown that ATA may act as an inhibitor for in vitro protein synthesis (Grollmann, A. P. and Stewart, M. L., Biochemistry 61 (1968) 719-725), or may act as an inhibitor polymerase activity of E.coli RNA polymerase and Q-β-replicase (Blumenthal, T. and Landers, T. A., Biochem Biophys Res Commun 55 (1973) 680-8). It has also been shown that partial fractions of ATA are inhibiting reverse transcriptase activity of Mouse Maloony Leucemia Virus (Givens, J. F. and Manly, K. F., Nucleic Acids Res 3 (1976) 405-18).

Inhibition of RNA and DNA polymerase activities has been demonstrated for formaurin tricarboxylic acid, whereas pure aurintricarboxylic acid in contrast to the commercially available heterogeneous solution shows no activity (Tsutsui, K., et al., Biochim Biophys Acta 517 (1978) 14-23). Moreover, stimulation of transcription in isolation of isolated nuclei which was presumably due to removal of histones and other proteins from the chromatin has also been described (Swennen, L., et al., Arch Int Physiol Biochim 86 (1978) 925-6).

In addition to the inhibition of nucleic acid polymerases, it has also been shown that commercially available polymeric ATA inhibits RNAse A by means of binding to the catalytic site of the protein (Gonzalez, R. G., et al., Biochemistry 19 (1980) 4299-303). Based on these studies, polymeric ATA has been used as an RNAse inhibitor for transcription studies (Schulz-Harder, B. and Tata, J. R., Biochem Biophys Res Commun 104 (1982) 903-10) and isolation of cellular RNA (Skidmore, A. F. and Beebee, T. J., Biochem J 263 (1989) 73-80; Williams, C. and Krawisz, B., Methods in Molecular & Cellular Biology 1 (1989) 35-40).

ATA may also act as an inhibitor of DNA binding of transcription factors such as NF-kappaB (Sharma, R. K., et al., Bioorg Med Chem 8 (2000) 1819-23). Furthermore, it has been shown that ATA has an antiviral effect due to binding to the CD4 surface receptor as well as due to its high affinity to the GP120 protein of the HIV virus (Cushman, M., et al., Journal of Medicinal Chemistry 34 (1991) 329-337; Neurath, A. R., et al., Antiviral Chemistry & Chemotherapy 2 (1991) 303-312).

A major problem with nucleic acid amplification and more especially with PCR is the generation of nonspecific amplification products. In many cases, this is due to an nonspecific oligonucleotide priming and subsequent primer extension event prior to the actual thermocycling procedure itself, since thermostable DNA polymerases are also moderately active at ambient temperature. For example, amplification products due to by chance occurring primer dimerisation and subsequent extension are observed frequently.

In order to overcome this problem, it is well known in the art to perform a so called "hot start" PCR, wherein one component essential for the amplification reaction is either separated from the reaction mixture or kept in an inactive state until the temperature of the reaction mixture is being raised for the first time. Since the polymerase cannot function under these conditions, there is no primer elongation during the period when the primers can bind non-specifically. In order to achieve this effect, several methods have been applied:

### a) Physical separation of the DNA polymerase

The physical separation can be obtained for example by a barrier of solid wax, which separates the compartment containing the DNA polymerase from the compartment containing the bulk of the other reagents. During the first heating step the wax is then melting automatically and the fluid compartments are mixed (Chou, Q., et al., Nucleic Acids Res 20 (1992) 1717-23). Alternatively, the DNA polymerase is affinity immobilized on a solid support prior to the amplification reaction and only released into the reaction mixture by a heat mediated release (Nilsson, J., et al., Biotechniques 22 (1997) 744-51). Both methods, however are time consuming and inconvenient to perform.

### b) Modification of DNA polymerase

For this type of hot start PCR, the DNA polymerase is reversibly inactivated as a result of a chemical modification. More precisely, heat labile blocking groups are introduced into the Taq DNA polymerase which render the enzyme inactive at room temperature. These blocking groups are removed at high temperature during a pre-PCR step such that the enzyme is becoming activated. Such a heat labile modification, for example can be obtained by coupling citraconic anhydride or aconitic anhydride to the lysine residues of the enzyme (US 5,677,152). Enzymes carrying such modifications are meanwhile commercially availabile as Amplitaq Gold (Moretti, T., et al., Biotechniques 25 (1998) 716-22) or FastStart DNA polymerase (Roche Molecular Biochemicals).

US 6,183,998 discloses the use of aldehydes for reversible modification of thermostable enzymes. However, the introduction of blocking groups is a chemical reaction which arbitrarily occurs on all sterically available lysine residues of the enzyme. Therefore, the reproducibility and quality of chemically modified enzyme preparations may vary and can hardly be controlled.

In addition, it has also been disclosed that a cold sensitive polymerase mutant (US 6214557) with decreased activity at 25 °C was found to result in more specific PCR product

### c) Taq DNA antibodies

An alternative approach to achieve heat labile inhibition of Taq DNA polymerase is the addition of monoclonal antibodies raised against the purified enzyme (Kellogg, D. E., et al., Biotechniques 16 (1994) 1134-7; Sharkey, D. J., et al., Biotechnology (N Y) 12 (1994) 506-9). Like the oligonucleotide aptamers, the antibody binds to Taq DNA polymerase with high affinity at ambient temperatures in an inhibitory manner. The complex is resolved in a preheating step prior to the thermocycling process itself. This leads to a substantial time consuming prolongation of the amplification as a whole, especially if protocols for rapid thermocycling are applied (WO 97/46706).

US 5,985,619 discloses a specific embodiment for performing PCR using a hot start antibody, wherein besides Taq polymerase, e. g. Exonuclease III from E. *coli* is added as a supplement to the amplification mixture in order to digest unspecific primer dimer intermediates. As disclosed above, Exonuclease III recognizes double stranded DNA as a substrate, like, for example, target/primer- or target/primer extension product hybrids. Digestion is taking place by means of cleavage of the phosphodiester bond at the 5' end of the 3' terminal deoxynucleotide residue.

Since this type of exonuclease is active at ambient temperatures, all nonspecifically annealed primers and primer extension products therefore are digested. This results in some embodiments in an even enhanced specifity of the amplification reaction. Yet, digestion of the nonspecific primers dependent on the duration of the preincubation time may lead to a substantial and uncontrolled decrease in primer concentration, which in turn may affect the amplification reaction itself.

### d) Usage of Exonucleases

Another alternative for increasing amplification efficiency is the use of phosphorothioate oligonucleotide primers in combination with an exonuclease III in the PCR reaction mixes (EP 0 744 470). In this case, a 3' exonuclease, which usually accepts double stranded as well as single stranded DNA substrates, degrades duplex artefacts such as primer dimers as well as carry over amplicons, while leaving the single stranded amplification primers undegraded. Similarly, the usage of primers with abasic modified 3' ends and template dependent removal by *E.coli* Endonuclease IV has been suggested (US 5,792,607).

However, there exists a major draw backs of this methods: phosphorthioate primers could not be prepared in a stereoisomeric pure manner and the method requires enzym preparation and purification.

### e) modified primers

EP 0 799 888 and GB 2293238 disclose an addition of 3' blocked oligonucleotides to PCR reactions. Due to the 3' block, these oligonucleotides can not act as primers. The blocked oligonucleotides are designed to compete/interact with the PCR primers which results in reduction of non-specific products.

Primers with an elongated 5' end are used to avoid primer dimers by formateion of panhandles (Brownie, J., et al., Nucleic Acids Res 25 (1997) 3235-41). Similarly, primers with self-complementary regions are non linear and could not act as primer prior to heating (Kaboev, O. K., et al., Nucleic Acids Res 28 (2000) E94; Ailenberg, M. and Silverman, M., Biotechniques 29 (2000) 1018-20, 1022-4). These two approaches, however, result in extremely more difficult primer design.

EP 0 866 071 describes the use of primers which are modified by bulky groups which are attached to the exocyclic amino groups of the nucleobases. Therefore, hybridization capability of these primers is significantly reduced. Upon heating, the interference with Watson Crick base pairing is reduced and extension of the primer occurs. Alternatively photoremovable groups may be attached to the exocyclic amino groups of the nucleobases. Heating at high temperatures then results in formateion of the unmodified primer.

WO 0043544 discloses the use of primers which have been modified with haptens like fluorescein in combination with an anti-fluorescein antibody. The formed immuno-complex prevents nonspecific PCR at low temperatures, but is disrupted at higher temperatures. Due to the required chemical modifications, however, these alternatives are considerably time and cost extensive.

### f) Nucleic acid additives

Extension of non-specifically annealed primers has been shown to be inhibited by the addition of short double stranded DNA fragments (Kainz, P., et al., Biotechniques 28 (2000) 278-82). In this case, primer extension is inhibited at temperatures below the melting point of the short double stranded DNA fragment, but independent from the sequence of the competitor DNA itself. However, it is not known, to which extent the excess of competitor DNA influences the yield of the nucleic acid amplification reaction.

Alternatively, oligonucleotide aptamers with a specific sequence resulting in a defined secondary structure may be used. Such aptamers have been selected using the SELEX Technology for a very high affinity to the DNA polymerase (US 5,693,502), (Lin, Y. and Jayasena, S. D., J Mol Biol 271 (1997) 100-11). The presence of such aptamers within the amplification mixture prior to the actual thermocycling process itself again results in a high affinity binding to the DNA polymerase and consequently a heat labile inhibition of its activity.

Due to the selection process, however, all so far available aptamers can only be used in combination with one particular species of DNA polymerase.
Oligonucleotide inhibitors (WO 0102559) with blocked 3' end and 5' end were shown to enhance sensitivity and specifictiy of PCR reactions. They compete with the primer in binding to the polymerase.

### g) other PCR additives

Other organic additives known in the art like DMSO, betaines, and formamides (WO 99/46400, (Hengen, P. N., Trends Biochem Sci 22 (1997) 225-6; Chakrabarti, R. and Schutt, C. E., Nucleic Acids Res 29 (2001) 2377-81) result in an improvement of amplification of GC rich sequences, rather than prevention of primer dimer formateion. Similiarily, heparin may stimulate in vitro run-on transcription presumably by removal of proteins like histones in order to make chromosomal DNA accessible (Hildebrand, C. E., et al., Biochimica et Biophysica Acta 477 (1977) 295-311).

It is also known that addition of single strand binding protein (US 5,449,603) or tRNA, (Sturzenbaum, S. R., Biotechniques 27 (1999) 50-2) results in non-covalent association of these additives to the primers. This association is disrupted when heating during PCR. It was also found that addition of DNA helicases prevent random annealing of primers (Kaboev, O. K., et al., Bioorg Khim 25 (1999) 398-400). Furthermore, poly-glutamate (WO 00/68411) in several cases may be used in order to inhibit polymerase activity at low temperatures.

Unfortunately, preparation of all of these different biological compounds requires costly and time consuming biochemical purification procedures.

Thus , there is a need in the art to provide an alternative hot start PCR method, which is cheap, easy to perform, and - most important - which does not affect the performance of the PCR reaction itself.

### Brief description of the invention

The present invention provides a new alternative solution for comfortable hot start PCR.

This method is based on the inventor's observation that in contrast to commercially available ATA which is composed of a mixture of poly-hydroxy-aryl-poly-acid monomers, oligomers and polymers, certain defined low molecular weight poly-hydroxy-aryl-poly-acids surprisingly improve the perfomance of an amplification reaction, if they are added in pure form to a nucleic acid amplification mixture.

Therefore, in a first aspect, the invention is directed to the use of a poly-hydroxy-aryl-poly-acid with 3-6 ortho-hydroxy-acid moieties as an additive for a nucleic acid amplification reaction. Addition of such a compound provides a means in order to enhance the sensitivity of the amplification reaction.

More specifically, the invention is directed to the use of a compound with 3-6 ortho-hydroxy-acid moieties as an additive for a nucleic acid amplification reaction, wherein said compound has the formula 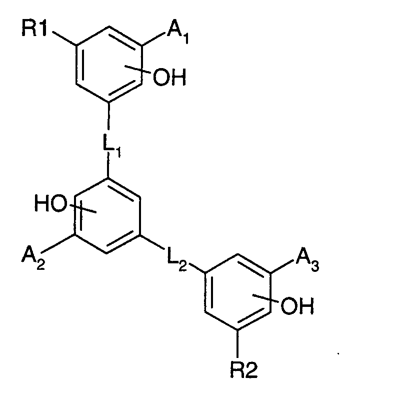 characterized in that
A1, A2, A3 are either identical or different and each represent an acid moiety
the - OH groups are in an ortho position with respect to A1, A2 and A3
R1, R2 are either identical or different and each represent either H or lower alkyl,CH₂-Aryl, a halogen or an acid moiety
L1, L2 are either identical or different and each represent either
-CH₂-,
-CO-,
- CR3 (O-A)-, wherein A is H, a lower alkyl, or aryl
- CR3(N(B1B2))-, wherein B1 and B2 are either H or a lower alkyl, or aryl
or - C⁺aryl,
characterized in that R3 is either H or aryl.

Preferably, the nucleic acid amplification reaction is a Polymerase Chain Reaction (PCR). Even more preferably, the amplification reaction is a Real Time PCR, wherein generation of the amplification product is monitored by means of fluorescence in real time.

In a second aspect, such a poly-hydroxy-aryl-poly-acid may generally be used as an additive for a mixture containing nucleic acids in order to decrease nucleotide base pairing interactions thus preventing nonspecific hybridization effects.

In a third aspect, a poly-hydroxy-aryl-poly-acid may be used as an additive for determination of nucleic acid melting points, i. e. determination of melting temperatures at which an at least partially double stranded nucleic acid hybrid molecule denatures into two single stranded molecules.

In yet another aspect, the new invention is directed to a composition comprising components necessary to perform a nucleic acid reaction and the disclosed poly-hydroxy-aryl-poly-acid. Similarly, the invention is directed to a kit comprising a composition with all components necessary to perform a nucleic acid reaction and a poly-hydroxy-aryl-poly-acid as disclosed above.

### Detailed description of the invention

As indicated above, it is an object of the present invention to provide an improved method for hot start nucleic acid amplification. According to the invention, improved nucleic acid amplification is achieved by means of using a poly-hydroxy-aryl-poly-acid with 3-6 ortho-hydroxy-acid moieties. In other words, the acid substituents and the hydroxyl substituents each are attached to an aromatic ring at adjacent carbon atoms.

Addition of such a compound enhances the sensitivity of the amplification reaction. Preferably, the nucleic acid amplification reaction is a Polymerase Chain Reaction, using a forward and a reverse primer, a thermostable DNA Polymerase and nucleoside triphosphates in order to generate an amplification product during an adjusted thermocycling protocol comprising a primer annealing phase, a primer elongation phase and a denaturation phase.

Also preferably, the compound according to the invention is added to the amplification mixture in the form of a salt, preferably with a monovalent or divalent cation, and most preferred as an ammonium salt. It is also noted that according to the invention, a poly-hydroxy-aryl-poly-acid with 3-6 ortho-hydroxy-acid moieties is present in an amplification mixture in a final concentration range of 0,001- 1 mg/ml in order to generate a hot start effect. Preferably, the additive according to the invention is present at final concentrations between 0,01 and 0,1mg/ml. The exact final concentration optimum, however, may vary dependent on the additive compound that is used and also depending on the nature of the other compounds of the amplification mixture.

It is also within the scope of invention, if a poly-hydroxy-aryl-poly-acid with 3-6 ortho-hydroxy-acid acid moieties is added to an RT-PCR reaction, characterized in that an RNA template sequence is amplified. RT-PCR can be performed according to different protocols, wherein first strand and second strand synthesis are either performed by two different enzymes or by using the same enzyme for both steps.

For the first strand synthesis, any kind or RNA dependent DNA Polymerases (reverse transcriptase) may be used, e.g. AMV Reverse Tanscriptase or MMLV Reverse Transcriptase. Subsequently, the RNA/cDNA hybrid is subjected to thermal denaturation or alternatively, the RNA template within the hybrid may be digested by RNaseH. Second strand synthesis and amplification may then be performed in the presence of an additive with any kind of thermostable DNA dependent DNA Polymerase like e.g. Taq DNA Polymerase according to standard protocols.

Alternatively, for an approach where first and second strand synthesis is done with one enzyme, thermostable DNA polymerases like Thermus thermophilus or C.Therm (Roche Diagnostics) may be used in the presence of an additive according to the invention.

In one specific embodiment, the disclosed poly-hydroxy-aryl-poly-acid is used as an additive to a Real Time PCR amplification mixture, wherein amplification is monitored in real time. Usually, this is enabled by means of fluorescence.

For example, fluorescently labeled hybridization probes may be used for real time monitoring. Those hybridization probes used for the inventive method according to the invention are usually single-stranded nucleic acids such as single-stranded DNA or RNA or derivatives thereof or alternatively PNAs which hybridize at the annealing temperature of the amplification reaction to the target nucleic acid. These oligonucleotides usually have a length of 20 to 100 nucleotides.

The labeling can be introduced on any ribose or phosphate group of the oligonucleotide depending on the particular detection formate. Labels at the 5' and 3' end of the nucleic acid molecule are preferred.

The type of label must be detected in the real-time mode of the amplification reaction. This is not only possible for fluorescently labeled probes but also possible with the aid of labels that can be detected by NMR. Methods are particularly preferred, however in which the amplified nucleic acids are detected with the aid of at least one fluoresent-labelled hybridization probe.

Many different test procedures are possible. The following 4 detection formates have proven to be particularly useful in connection with the use of an additive of the invention, but shall not be understood as limiting the inventive scope.

### (i) FRET hybridization probes

For this test formate two single-stranded hybridization probes are used simultaneously which are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.

Among all detection formates possible within the scope of the present invention, this "FRET-hybridization probe" has been proven to be highly sensitive, exact and reliable.

Alternatively, it is also possible to use a fluorescent-labeled primer and only one labeled oligonucleotide probe (Bernard et al., Analytical Biochemistry 235, p. 1001-107 (1998)).

### (ii) TaqMan hybridization probes

A single-stranded hybridization probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured.

### (iii) Molecular Beacons

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### (iv) SybrGreenI formate

It is also within the scope of the invention, if real time PCR is performed in the presence of an additive according to the invention in case the amplification product is detected using a double stranded nucleic acid binding moiety. For example, the respective amplification product can also be detected according to the invention by a fluorescent DNA binding dye which emits a corresponding fluorescence signal upon interaction with the doublestranded nucleic acid after excitation with light of a suitable wavelength. The dyes SybrGreenI and SybrGold (Molecular Probes) have proven to be particularly suitable for this application. Intercalating dyes can alternatively be used. However, for this formate, in order to discriminate the different amplification products, it is necessary to perform a respective melting curve analysis (US 6,174,670).

In another embodiment of the invention which may also include real time monitoring, the amplification reactions are performed in the presence of an additive according to the invention by means of rapid thermocycling, wherein one cycle is less than one minute. The instrumental basis for such a rapid theromocycling is provided e.g. by the LightCycler (Roche Molecular Biochemicals) and disclosed in WO 97/46707 and WO 97/46712. According to the present invention, the annealing of primers is not the rate limiting step of quantitative amplification of target nucleic acids being present in the sample with only low abundance. Therefore, the time parameters for a respective multiplex thermocycling protocol in order to amplify low abundance parameters don't need to be amended as compared to any kind of thermocycling protocols known in the art.

It is also within the scope of the new invention, if, a poly-hydroxy-aryl-poly-acid with 3-6 ortho-hydroxy-acid moieties is used as an additive for a mixture containing nucleic acids in order to decrease nonspecific hybridization effects. Besides its positive effect on prevention of primer dimer formateion in a hot start PCR approach, addition of such a poly-hydroxy-aryl-poly-acid also may enhance specificity of any kind of analytical hybridization method like, e.g. Southern blot analysis, Northern Blot analysis, Dot blot analysis e.c.

In a still other aspect of the invention, such a poly-hydroxy-aryl-poly-acid with 3-6 ortho-hydroxy-acid moieties may be used as an additive for determination of nucleic acid melting points, i. e. determination of melting temperatures at which an at least partially double stranded nucleic acid hybrid molecule denatures into two single stranded molecules. Preferably, it may be added for embodiments of real time PCR, characterized in that subsequent to the amplification reaction itself, the amplification product(s) are identified by a melting curve analysis. As will be shown in the examples, addition of a poly-hydroxy-aryl-poly-acid according to the invention results in a much sharper shape of melting curve peaks in 1^{st} derivative fluorescence versus temperature blots and thus in a more accurate determination of an exact melting temperature.

In yet another aspect, the new invention is directed to a composition comprising components necessary to perform a nucleic acid amplification reaction and a poly-hydroxy-aryl-poly-acid with 3-6 ortho-hydroxy-acid moieties. In one embodiment, the inventive composition is a generic composition, which comprises all necessary components for nucleic acid amplification but lacks specific amplification primers. In a second embodiment, the inventive composition is a parameter specific composition which comprises all components necessary to perform a specific amplification reaction including a specific pair of amplification primers. In a third embodiment, a composition according to the second embodiment additionally comprises detection moieties like suitable fluorescent hybridization probes or ds nucleic acid binding compounds such as SybrGreen.

Similarly, the invention is directed to a kit comprising a composition with all components necessary to perform a nucleic acid reaction and a poly-hydroxy-aryl-poly-acid with 3-6 ortho-hydroxy-acid moieties. Preferably, these kits comprise compositions as disclosed above. Alternatively, the components necessary for amplification and the additive according to the invention may be present in different storage vessels.

### Structure of the poly-hydroxy-aryl-poly-acid additives according to the invention

Many different poly-hydroxy-aryl-poly-acids with 3-6 ortho-hydroxy-acid moieties have been identified by the inventors to result in a hot start effect and thus a more effective nucleic acid amplification reaction. In particular, it has been proven to be advantageous, if the poly-hydroxy-aryl-poly acid according to the invention does not contain any additional acid moieties, i. e. if the overall number of acid moieties and hydroxyl moities are identical.

In particular, these compounds may have the following structure: 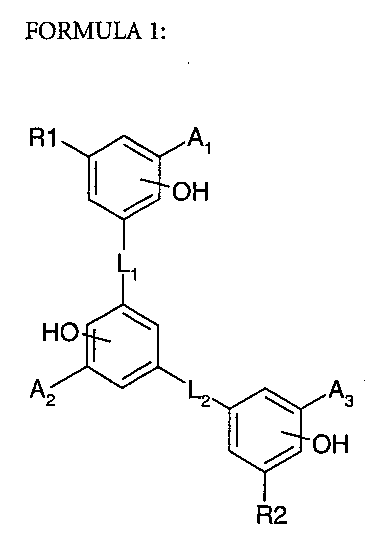 Characterized in that
A1, A2, A3 are either identical or different and each represent an acid moiety
the - OH groups are in an ortho position with respect to A1, A2 and A3
R1, R2 are either identical or different and each represent either H or lower alkyl or CH₂-aryl, a halogen, or an acid moiety
L1, L2 are either identical or different and each represent either
-CH₂-,
-CO-,
- CR3(O-A)-, wherein A is H, a lower alkyl, or aryl
- CR3(N(B1B2))-, wherein B1 and B2 are either H or a lower alkyl, or aryl
or - C⁺aryl-
characterized in that R3 is H or aryl.

"Lower alkyl" in this context shall mean an alkyl consisting out of 1-6 C-atoms.

Aryl moieties are aromatic ring structures consisting out of 6-22 C atoms. In view of the experimental synthesis requirements however, ring structures consisting out of 6 C-atoms are highly preferred.

Aryl moieties in the context of this invention include moities, which carry one or more additional substitutents. Such substituents, for example, may be halogens, hydroxyl-groups, acid groups or lower alkyl groups.

Use of poly-acids with heterocyclic aromatic ring structures consisting out of 5 C-atoms and an additional N-atom as a PCR additive is also within the scope of this invention. Consequently, "aryl" in the context of the invention shall preferentially mean an aromatic hexameric ring structure which may be a homocyclic ring of 6 C-atoms or, alternatively, a heterocyclic ring of 5 C-atoms and one N-atom such as a pyridine.

Furthermore, it is also within the scope of the new invention, if one, several or all aromatic ring structures of FORMULA 1 are replaced by heterocyclic hexameric ring structures consisiting out of 5 C-atoms and one N-atom.

Several of the compounds disclosed may also be in a quinoid form as it will be disclosed below.

The quantitatively observed hot start effects upon addition of different poly-hydroxy-aryl-poly-acids with 3-6 acid moieties, however, are different depending on the actual type of molecule which is used as additive. Thus, by comparison of the molecular structures of the different compounds tested, several features have been proven to be advantageous in order to gain the desired hot start effect:

First, it is advantageous if the number of aryl moieties is limited. Preferably, an additive according to the invention consists of 3 or 4 Aryl moieties, since the hydrophobic groups of larger molecules may result in nonspecific interactions with the protein components of an amplification reaction mixture.

For each aryl moiety, the acidic groups and hydroxyl groups may directly be bound to the aromatic ring thus forming a phenolic structure. Alternatively, the acidic groups may be either bound directly to the aromatic ring or alternatively be connected to the aromatic ring via a bridge of C-atoms preferably consisting only of 1, 2 or another small number of residues.

In a highly preferred embodiment, all hydroxyl groups and the acidic groups are directly bound to the aromatic ring and located in ortho position with respect to each other.

Second, although it is possible to have different acidic entities like for example carboxylic acids, sulfonic acids or phosphonic acids, it is advantageous if all acidic residues present in the additive according to the invention are identical. Moreover, it is also preferred if at least one, several or all acidic residues are carboxylic acids.

Third, the additives used according to the invention may be aurintricarboxylic acid derivatives, because - as it will be shown in the examples below - starting from salicylic acid and/or methylen-bi-salicylic acid, such derivatives can be produced with a reasonable moderate preparative effort.

It is a characteristic feature of the invention, that poly-hydroxy-aryl-poly-acids are used, for which at least three acid moieties are each connected with another acid moiety by a chain of 7 C-Atoms. The C-atoms themselves within the chain may be connected via a single covalent bond, a double bond or are preferably part of one or several aromatic ring systems. Although the advantage of such a structure is rather obvious from the experimental data generated by the inventors so far, the scientific explanation for this surprising effect still remains unclear.

In a specific embodiment (which is definitely not mutually exclusive to the feature disclosed above), the additive according to the invention is a compound as follows: 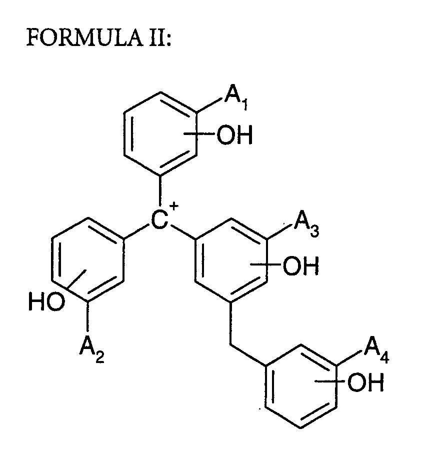 wherein A1, A2, A3 and A4 each denote an acid moiety, which may be identical or different. Again, A1, A2, A3 and A4 are preferably carboxylic acids. Moreover, it is essential that all hydroxyl groups are located in ortho position with respect to the corresponding carboxylic acid moiety of the same aromatic ring.

Compounds according to the invention also include deprotonated forms wherein the hydroxyl groups and/or the acidic groups are deprotonated. There is no limitation regarding the nature of the corresponding cation. However, ammonium, tri-/tetralakylammonium, sodium and magnesium (or monovalent and divalent) cations are preferred as counterions.

Compounds according to FORMULA I wherein one or both L1/L2 = C⁺aryl can also be disclosed by a formula which emphasizes the quinoid character of such compounds, since e.g. the quinoid structure and the triphenylmethinium structure according to FORMULA 2 are only two different ways to describing the same compound. In these cases, addition of nucleophiles like hydroxy anions, water, ammonia, mercaptanes to the methinium carbon atom - eventually followed by deprotonation - may occur. Such an addition, however, is known in the art to be reversible.

One specific representative which shows an extensive hot start effect when added to a PCR amplification mixture is shown below in FORMULA III:

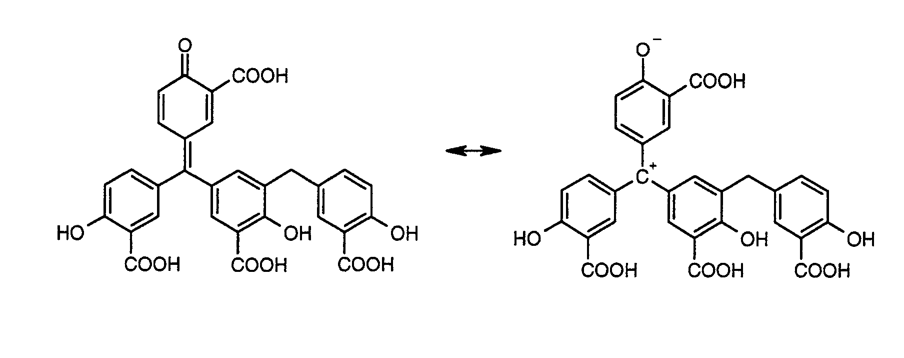

### Description of the Figures:

Figure 1: Inhibition of primer dimer formation in presence of compound I
   1a: Detection in real time PCR
   1b: Detection in subsequent gel electrophoresis
Fig. 2: Increase in real time PCR sensitivity in presence of compound I
   - SybrGreen formate
Fig. 3: Increase in real time PCR sensitivity in presence of compound I
   - FRET-HybProbe formate
   3a: 0, 4 fg plasmid containing a complete DPD cDNA
   3b: 4 fg plasmid containing a complete DPD cDNA
   3c: 40 fg plasmid containing a complete DPD cDNA
Fig. 4: Improvement of real time PCR melting curve analysis in presence of a compound I according to example 2

The following examples, references, sequence listing and figures are provided to aid the understanding of the present invention, the true scope of which is set forth in the appended claims. It is understood that modifications can be made in the procedures set forth without departing from the spirit of the invention.

### Examples:

### Example 1

### Synthesis of ATA

The ammonium salt of aurintricarbocyclic acid (ATA) was prepared by a modification of the procedure published by D.A. HOLADAY, J. Am. Chem.Soc. 62, p. 989. (1940). Briefly, 5 g of sodium nitrate (Aldrich 43,160-5) was added slowly under vigorous stirring to 36 cc. of sulfuric acid (Merck 731) at room temperature under argon atmosphere. To this solution an intimate mixture of 10 g (35 mmol) methylene-bisalicylic acid (ABCR, AV 12953) and 5 g (36 mmol) of salicylic acid (Aldrich 24,758-8) was added in small portions with vigorous stirring. After addition of all the solid materials the mixture was allowed to stand for 1 hour with occasional stirring. A mixture of 5 g sodium nitrate in 36 cc. of sulfuric acid prepared as described above was added to the brownish red solution and stirred overnight at room temperature. The reaction mixture was added slowly to 11 of ice-cold water, allowed to stand for an hour, and filtered on a Büchner funnel. The supernatant collected on the Büchner funnel was washed with deionized water until the filtrate was acid free. The supernatant was dried under vacuum over calcium chloride at 50 °C. The yield of the dried product was 12.5 g.

The preparation and purification of the different ammonium salts of the ATA derivatives was performed accordingly to M. CUSHMAN using ultrafiltration and silica gel chromatography (column and preparative thin layer chromatography) (J. Org. Chem. 57, 3861-3866 (1992) and J. Org.Chem. 57, 7241-7248 (1992).

### Example 2

### Ultrafiltration of low molecular weight ATA derivatives and purification of compound I according to FORMULA III

10.5 g of ATA raw product was dissolved in 11 of 0.1 N NaHCO₃ solution and loaded onto a MW 1000 Cutoff Membrane: A total of 4 l of ultrafiltrate was collected from three operations adding water step wise. The ultrafiltrate was evaporated to a volume of 300 ml, and the solution was acidified with HCl to precipitate the ATA compounds. The precipitate was collected by filtration and washed acid free with destilled water. After adding diluted ammoniumhydroxide the dissolved ATA-Ammonium salt was lyophilized (Yield: 4.6g).

1 g of the fractionated ATA sample was dissolved in acetone (250 ml) and added to silica gel 60 (50 g, Merck). The solvent was evaporated to dryness of the silica gel. The mixture was then placed on the top of a wet column of the same type of silica gel and the column was eluted with a solvent mixture of containing chloroform/formic acid/tetrahydrofuran (100:4:0.3). The column chromatography was monitored by TLC. The solvent mixture is stepwise changed to 100:6:2 and 100:6:10 (chloroform/formic acid/tetrahydrofurane) until the target substance is eluted, monitored by TLC with the same system.

Final purification of compound I was done by preparative TLC with the system chloroform/formic acid/tetrahydrofuran (100:6:2).

NMR spectra were measured with Bruker DPX 300. ¹H-NMR spectra were recorded at 300 MHz and ¹³C-NMR spectra were obtained at 75 MHz. The NMR samples were prepared in DMSO-d₆ and were referenced to TMS as an internal standard. ESI-MS was measured with Micromass Platform II using 0.02 % TFA in water/40% acetonitril.
UV: λₘₐₓ (Ethanol). 309 nm, 528 nm
TLC (silica gel 60; developed with isopropanol/25% ammonia/water 6:3:1): R_{f}: 0,75
¹H-NMR (300 MHz, DMSO-d₆)
7.60 (d, J=2.4 Hz, 2H), 7.57 (d, J=21 Hz, 1H), 7.49 (d, J=2.3 Hz, 1H), 7.27 (m, 5H), 6.88 (d, J=8.7 Hz, 2H), 6.82 (d, J=8.5 Hz, 1H), 3.96 (s. 2H)
¹³C-NMR (75 MHz, DMSO-d₆)
δ 173.20, 172.70, 172.66, 160.86, 160.29, 159.18, 139.05, 138.49, 136.57, 135.80, 131,64, 130.70, 129.74, 129.13, 127.87, 117.75, 117.29, 113.43, 112.99, 112.59, 79.90
ESI-MS: 573.0

### Example 3

### Inhibition of Primer Dimer Formation in a PCR

For this experiment, a 10 fold primer mix was prepared for amplification of a sequence from the Dihydropyrimidin-dehydrogenase (DPD) cDNA (cloned in a plasmid) consisting the following oligonucleotides (10µM each):

Primer DPD rev (Sequence:5'-TCT TGC GAT GCT CAC AAT ATC AGT GAC-3')
(Seq Id No.1)

Primer DPD fwd (Sequence :5'-ATG GCA TGG GAG AAA GAG GAA TG-3')
(Seq. Id. No.2)

Three serial single dilutions of the plasmid containing the cDNA isolated from a cell line constitutively expressing DPD were prepared with concentrations of c= 10 fg/µl, 1 fg/µl, 0.1 fg/µl in water. Independently, 10 mg of compound 1 synthesized and purified according to examples 1 and 2 were dissolved in 1 ml PCR-grade water.

PCR was carried out with LightCycler™ instrument (Roche Molecular Biochemicals) in the presence or absence of 0,1µg/µl compound according to Formula III from example 2 using the LC DNA Master SYBRGreen I Kit (Roche Molecular Biochemicals, Cat.No. 2158817-001, basically containing H₂O, PCR-grade, MgCl₂ (25 mM) and 10 x Mastermix for a 20 µl reaction.

Values for denaturation, amplification and melting curve analysis were programmed as follows:

| Program 1: Denaturation | |
|---|---|
| Cycle Program Data | Value |
| Cycles | 1 |
| Analysis Mode | None |

| ***Temperature Targets*** | ***Segment 1*** |
|---|---|
| Target temperature | 95 |
| Incubation time (min:s) | 30 |
| Temperature transition rate (°C/s) | 20 |
| Secondary target temperature (°C) | 0 |
| Step size (°C) | 0 |
| Step delay (cycles) | 0 |
| Acquisition mode | None |

| Program 2: Amplification | | | |
|---|---|---|---|
| Cycle Program Data | Value | | |
| Cycles | 40 | | |
| Analysis Mode | Quantification | | |

| ***Temperature Targets*** | ***Segment 1*** | ***Segment 2*** | ***Segment 3*** |
|---|---|---|---|
| Target temperature | 95 | 62 | 72 |
| Incubation time (s) | 0 | 10 | 10 |
| Temperature transition rate (°C/s) | 20 | 20 | 20 |
| Secondary target temperature (°C) | 0 | 0 | 0 |
| Step size (°C) | 0 | 0 | 0 |
| Step delay (cycles) | 0 | 0 | 0 |
| Acquisition mode | None | None | Single |

| Program 3: Melting curve | | | |
|---|---|---|---|
| Cycle Program Data | Value | | |
| Cycles | 1 | | |
| Analysis Mode | Melting curve | | |

| ***Temperature Targets*** | ***Segment 1*** | ***Segment 2*** | ***Segment 3*** |
|---|---|---|---|
| Target temperature | 95 | 65 | 95 |
| Incubation time (s) | 0 | 15 | 0 |
| Temperature transition rate (°C/s) | 20 | 20 | 0.1 |
| Secondary target temperature (°C) | 0 | 0 | 0 |
| Step size (°C) | 0 | 0 | 0 |
| Step delay (cycles) | 0 | 0 | 0 |
| Acquisition mode | None | None | Cont |

| Program 4: Cooling | |
|---|---|
| Cycle Program Data | Value |
| Cycles | 1 |
| Analysis Mode | None |

| ***Temperature Targets*** | ***Segment 1*** |
|---|---|
| Target temperature | 40 |
| Incubation time (min:s) | 30 |
| Temperature transition rate (°C/s) | 20 |
| Secondary target temperature (°C) | 0 |
| Step size (°C) | 0 |
| Step delay (cycles) | 0 |
| Acquisition mode | None |

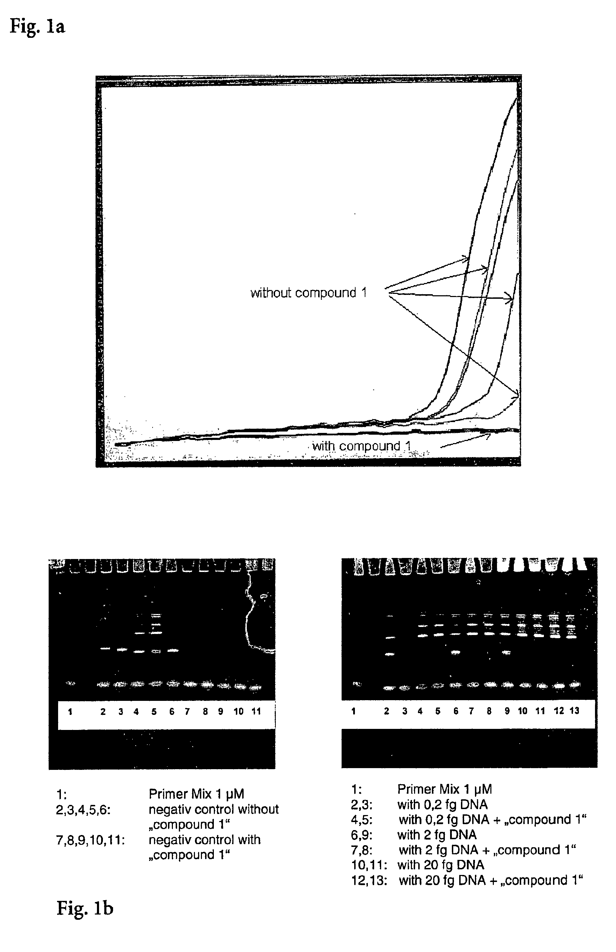

Reaction was monitored in real time using the SybrGreen modus of the LightCycler instrument according to the manufacturer's instructions. Fluorescent signalling of a sample containing a negative control without any target plasmid is shown in Fig. 1a. As can be seen in the figure a fluorescent signal corresponding to primer dimer formateion is only generated in the absence of a compound according to example 2.

Subsequently to the amplification, the content of the capillary reaction vessels were opened and the content was subjected to gel electrophoresis. In order to do so, he PCR-products were diluted with TBE-sample buffer 5x(Invitrogen LC6678/46-5022) and 7 µl were loaded onto a TBE Gel (Invitrogen EC63155) . After 70 min with 200 V the gel was stained with SYBRGreen II for 30 min and detected on the Lumi-Imager F1 (Roche Molecular Biochemicals, Cat. No. 2012847) (fluorescence: 520 nm, 20 sec).

Results are shown in Fig. 1b. As it can be seen in the figure, the fastest migrating band corresponds to single primers, the second lowest band to generated primer dimers and the upper bands correspond to specific amplification products. Addition of COMPOUND I (right gel, lines 4, 5, 7, 8, 12, and 13 constantly resulted in a complete inhibition of primer dimer formation even in those cases where only small amounts of template DNA (0,2 fg) were amplified.

### Example 4

### Increase in real time PCR sensitivity - SybrGreen formate

For this experiment, the LightCycler - Control Kit DNA (Roche Molecular Biochemicals, Catalog number 2158833 containing a Beta Globin Primer Mix (10 x 5µM each) and Control DNA consisting of human genomic DNA (15 ng/µl) was used in order to provide an amplification mixture containing 3pg/20µl DNA.

Primers were 
CAA CTT CAT CCA CGT TCA CC

(Seq. Id. No: 3)
 and

ACA CAA CTG TGT TCA CTA GC

(Seq.Id.No: 4)

PCR was performed in the presence or absence of a compound according to example 2 basically as disclosed for example 3 with the exception that for efficient ampification, an annealing temperature (target temperature) of 55°C instead of 62°C was used.

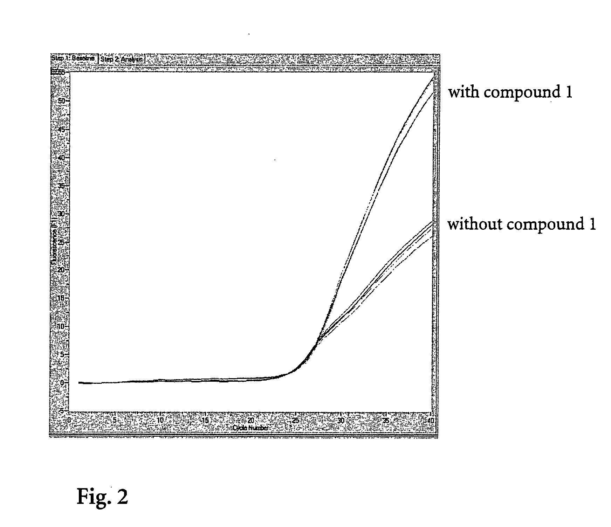

Results are shown in fig. 2. As can be seen in the figure, an enhancement of the signal up to 86% for cycle number above 30.

### Example 5

### Increase in real time PCR signal intensity - FRET-HybProbe formate

For this experiment, the primer mix according to example 3 was used. Various amounts of plasmid containing the complete DPD cDNA ranging from 0,4 to 40 fg were amplified with the primer mix by means of PCR under the conditions of example 3 in the presence or absence of 0,1µg/µl compound III according to example 2.

For Real Time PCR detection, FRET-hybridization probes from the LightCycler Dihydropyrimidin-Dehydrogenase (DPD) mRNA quantification kit (Roche Molecular Biochemicals, cat. No. 3136957) were used in concentrations according to the manufacturer's instructions.

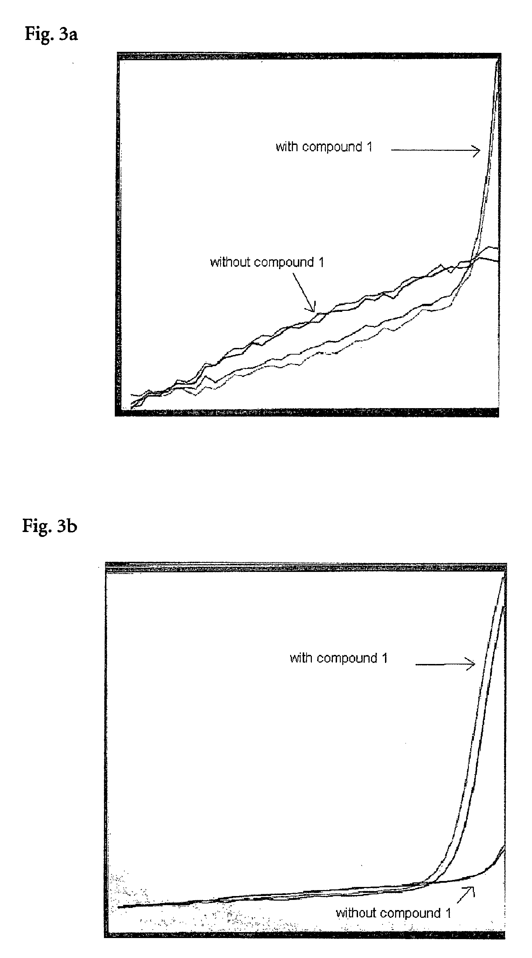

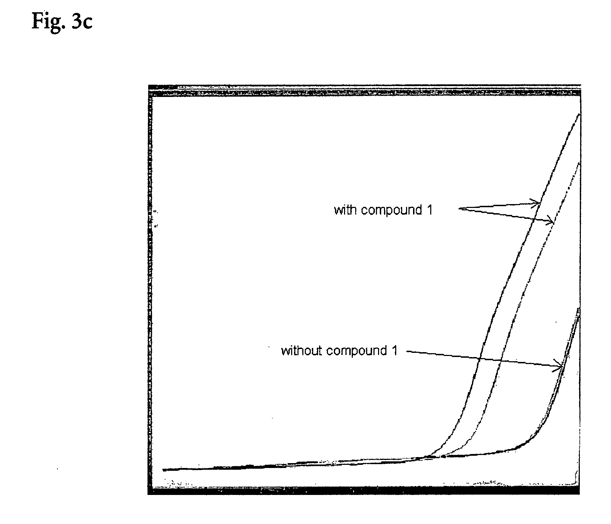

Results are shown in figure 3a-c. As can be seen in the figure 3a, with 0,4 fg template DNA, a specific amplification signal can only be obtained in presence of the additive according to the invention. In case of higher template concentrations (fig. 3b,c) addition of a compound according to the invention results in a specific signaling which can be detected at earlier cycle numbers.

### Example 6

### Improvement of melting curve analysis according to the invention

For this experiment, the LightCycler the LC DNA Master SYBR Green I Kit (Roche Molecular Biochemicals, Cat.No. 2158817-001 was used. A beta-actin primer mix (10 x 5µM each) was used for amplification of a respective fragment from human genomic DNA being present in the amplification mixture at a final concentration of 30ng/20µl DNA. Primers were: 
5′-TCA CGA TGC CAG TGG TAC GG
(Seq. Id. No: 5)
 and

5′-GGA TTC ATA TGT GGG GCA TG
(Seq. Id. No. 6)

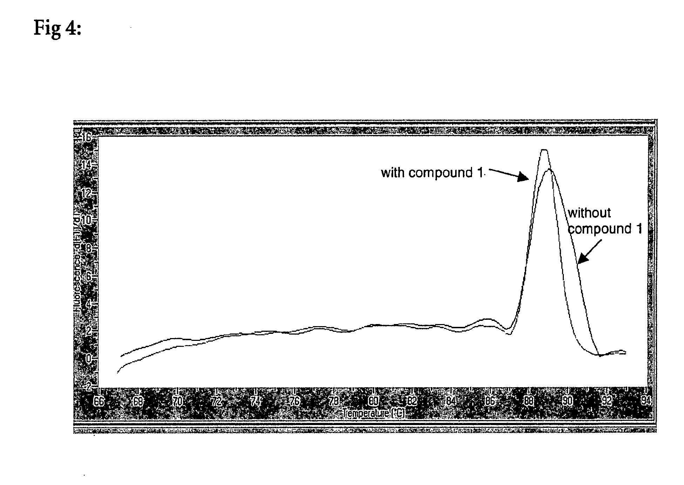

PCR was carried out in the presence or absence of a compound according to example 2 using LightCycler SYBR-Green I Formate basically according to example 3 and 4 with the modification that an annealing (target ) temperature of 60°C was applied. Subsequent to the amplification reaction, a melting curve analysis was performed on the LightCycler instrument (Roche Molecular Biochemicals) according to the manufacturer's instructions. The result of the melting curve analysis is shown in fig. 4. As can be seen in the figure, addition of a compound purified according to example 2 results in a much sharper melting peak as compared to the control reaction without a compound according to example 2.

### List of References

Ailenberg, M. and Silverman, M., Biotechniques 29 (2000) 1018-20, 1022-4
Blumenthal, T. and Landers, T. A., Biochem Biophys Res Commun 55 (1973) 680-8
Brownie, J., et al., Nucleic Acids Res 25 (1997) 3235-41
Chakrabarti, R. and Schutt, C. E., Nucleic Acids Res 29 (2001) 2377-81
Cushman, M., et al., J Med Chem 34 (1991) 337-42
Cushman, M., et al., Journal of Organic Chemistry 57 (1992) 7241-7248
Cushman, M., et al., Journal of Medicinal Chemistry 34 (1991) 329-337
Chou, Q., et al., Nucleic Acids Res 20 (1992) 1717-23
Givens, J. F. and Manly, K. F., Nucleic Acids Res 3 (1976) 405-18
Gonzalez, R. G., et al., Biochemistry 19 (1980) 4299-303
Grollmann, A. P. and Stewart, M. L., Biochemistry 61 (1968) 719-725
Hengen, P. N., Trends Biochem Sci 22 (1997) 225-6
Hildebrand, C. E., et al., Biochimica et Biophysica Acta 477 (1977) 295-311
Holaday, D. A., J. Am. Chem.Soc 68 (1940) 989
Kaboev, O. K., et al., Bioorg Khim 25 (1999) 398-400
Kaboev, O. K., et al., Nucleic Acids Res 28 (2000) E94
Kainz, P., et al., Biotechniques 28 (2000) 278-82
Kellogg, D. E., et al., Biotechniques 16 (1994) 1134-7
Lin, Y. and Jayasena, S. D., J Mol Biol 271 (1997) 100-11
Moretti, T., et al., Biotechniques 25 (1998) 716-22
Neurath, A. R., et al., Antiviral Chemistry & Chemotherapy 2 (1991) 303-312
Nilsson, J., et al., Biotechniques 22 (1997) 744-51
Schulz-Harder, B. and Tata, J. R., Biochem Biophys Res Commun 104 (1982) 903-10
Sharkey, D. J., et al., Biotechnology (N Y) 12 (1994) 506-9
Sharma, R. K., et al., Bioorg Med Chem 8 (2000) 1819-23
Skidmore, A. F. and Beebee, T. J., Biochem J 263 (1989) 73-80
Sturzenbaum, S. R., Biotechniques 27 (1999) 50-2
Swennen, L., et al., Arch Int Physiol Biochim 86 (1978) 925-6
Tsutsui, K., et al., Biochim Biophys Acta 517 (1978) 14-23
Williams, C. and Krawisz, B., Methods in Molecular & Cellular Biology 1 (1989) 35-40
EP 0 744 470
EP 0 799 888
EP 0 866 071
GB 2293238
US 5,118,801
US 5,449,603
US 5,677,152
US 5,693,502
US 5,792,607
US 5,985,619
US 6,183,998
US 6,174,670
WO 00/43544
WO 00/68411
WO 01/02559
WO 97/46706
WO 97/46707
WO 97/46712
WO 99/46400

## Claims

1. Use of a poly-hydroxy-aryl-poly-acid with 3-6 ortho-hydroxy-acid moieties as an additive for a nucleic acid amplification reaction.

2. Use of a compound with 3-6 ortho-hydroxy-acid moieties as an additive for a nucleic acid amplification reaction, wherein said compound has the formula 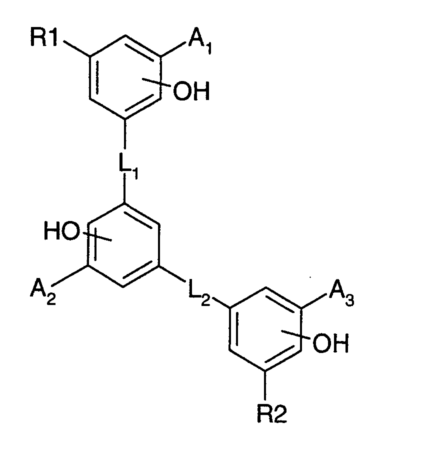 **characterized in that**
A1, A2, A3 are either identical or different and each represent an acid moiety
the - OH groups are in an ortho position with respect to A1, A2 and A3
R1, R2 are either identical or different and each represent either H or lower alkyl or CH₂-aryl, a halogen, or an acid moiety
L1, L2 are either identical or different and each represent either
-CH₂-,
-CO-,
- CR3(O-A)-, wherein A is H, a lower alkyl, or Aryl
- CR3(N(B1B2))-, wherein B1 and B2 are either H or a lower alkyl, or aryl
or - C⁺aryl-
wherein R3 is H or aryl

3. Use of a compound according to claim 1 or 2 as an additive to decrease nucleotide base pairing interactions.

4. Use according to claim 1 or 2, wherein addition of said compound enhances sensitivity of said amplification reaction.

5. Use according to claim 4, wherein said amplification is a real time PCR.

6. Use of a compound according to claim 1 or 2 as an additive for a nucleic acid melting point determination.

7. A composition comprising all components necessary to perform a nucleic acid reaction and a compound according to claim 1 or 2.

8. A kit comprising a composition with components necessary to perform a nucleic acid reaction and a compound according to claim 1 or 2.


SEQUENCE LISTING

<110> Roche Diagnostics GmbH
F. Hoffmann-La Roche AG

<120> Reagent for improved PCR

<130> 20977EP

<140>
<141>

<160> 6

<170> PatentIn Ver. 2.1

<210> 1
<211> 27
<212> DNA
<213> Artificial Sequence

<220>
<223> Description of Artificial Sequence:primer

<400> 1
tcttgcgatg ctcacaatat cagtgac 27

<210> 2
<211> 23
<212> DNA
<213> Artificial Sequence

<220>
<223> Description of Artificial Sequence:primer

<400> 2
atggcatggg agaaagagga atg                    23

<210> 3
<211> 20
<212> DNA
<213> Artificial Sequence

<220>
<223> Description of Artificial Sequence:primer

<400> 3
caacttcatc cacgttcacc                    20

<210> 4
<211> 20
<212> DNA
<213> Artificial Sequence

<220>
<223> Description of Artificial Sequence:primer

<400> 4
acacaactgt gttcactagc                    20

<210> 5
<211> 20
<212> DNA
<213> Artificial Sequence

<220>

<223> Description of Artificial Sequence:primer

<400> 5
tcacgatgcc agtggtacgg                                20

<210> 6
<211> 20
<212> DNA
<213> Artificial Sequence

<220>
<223> Description of Artificial Sequence:primer

<400> 6
ggattcatat gtggggcatg                                20